# EUROPÄISCHE PATENTSCHRIFT

(11) **EP 0 714 635 B1**
(45) Veröffentlichungstag und Bekanntmachung des Hinweises auf die Patenterteilung: **12.06.2002**
(21) Anmeldenummer: 95118244.3
(22) Anmeldetag: 20.11.1995
(51) Int. Cl.: A61B 18/12

(54) **Hochfrequenztherapieeinrichtung zur interstitiellen Thermotherapie von Tumoren**
High frequency device for interstitial thermotherapy of tumors
Appareil à haute fréquence pour la thermothérapie interstitielle des tumeurs

(30) Priorität: 30.11.1994 DE 4442690
(43) Veröffentlichungstag der Anmeldung: 05.06.1996
(73) Patentinhaber: Berchtold Holding GmbH, 78532 Tuttlingen (DE)
(72) Erfinder: Haendle, Hans, Dr. med., D-78532 Tuttlingen (DE); Müller, Wolfgang, Dr.-Ing., D-78532 Tuttlingen (DE); Wax, Alwin, D-78532 Tuttlingen (DE)
(74) Vertreter: Manitz, Finsterwald & Partner

(56) Entgegenhaltungen:
- WO-A-94/17856
- US-A- 4 932 952
- US-A- 5 122 138
- US-A- 5 197 963
- US-A- 5 348 555

## Beschreibung

Die Erfindung betrifft eine Hochfrequenz-Therapieeinrichtung nach dem Oberbegriff des Patentanspruchs 1 (vgl. WO-A-94/17856).

Hochfrequenzchirurgische Koagulations- und/oder Schneidvorrichtungen mit Spüllösungszufuhrrohren bzw. -schläuchen sind in verschiedenen Ausführungen bekannt (US-A-5122138, DE 39 21 000 A1, DE-U-87 07 820).

Es ist auch schon bekannt (DE AS 1 007 960), an einem mit Hochfrequenz beschickten Elektrodenkopf seitlich Spüllösungsaustrittsöffnungen vorzusehen, um bei der Koagulation austretendes Blut fortzuspülen und damit klare Sicht durch die Optik eines Endoskops zu gewährleisten.

Die Erfindung hat eine interstitielle Hochfrequenz-Therapieeinrichtung im Frequenzbereich von 300 kHz bis 1MHz mit Flüssigkeitszufuhr zum Behandlungsgebiet zum Gegenstand. Die Aufgabe der Erfindung besteht darin, nicht nur eine gleichmäßige Wärmebeaufschlagung des Biogewebes um die Arbeitsspitze herum im Interstitium zu gewährleisten, sondern auch ein möglichst minimalinvasives Einstechen der Einrichtung durch gesundes Gewebe hindurch zum Zielgebiet (Tumor, Hyperplasie) zu ermöglichen und ein Anhaften sowie Austrocknen von Biogewebe an der Arbeitsspitze zu vermeiden.

Zur Lösung dieser Aufgabe sind die Merkmale des kennzeichnenden Teiles des Anspruches 1 vorgesehen.

Durch das Vorsehen einer Metallspitze und dahinter angeordneter Spüllösungsaustrittsöffnungen wird bei Beaufschlagung der Arbeitselektrode mit Hochfrequenz nach dem Einstechen der Arbeitsspitze in Gewebe gewährleistet, daß durch Spülung mit einer Spüllösung unmittelbar um die Metallspitze herum ein Anhaften von Biogewebe an der Elektrode und ein thermisches Austrocknen vermieden wird. Die Spüllösung kann auch zu einer besseren Wärmeverteilung und Wärmeübertragung an das Biogewebe beitragen, wenn sie elektrisch leitfähig ist.

Die Ausführungsformen nach den Ansprüchen 2 bis 4 sind besonders vorteilhaft, weil durch die Hintereinanderschaltung von Spüllösungsaustrittsöffnungen im Innenrohr und der Axialschlitze im Elektrodenrohr eine Flüssigkeitskaverne geschaffen wird, die in Verbindung mit einem teflonisierten Schaft für ein besonders gutes axiales Gleiten des Instrumentes im Biogewebe und zu einer optimalen Wärmeübertragung führt.

Vorteilhafte bauliche Ausführungen sind durch die Ansprüche 5 bis 7 gekennzeichnet.

Eine optimale Therapie kann durch die zusätzliche Temperaturmessung gemäß Anspruch 8 gewährleistet werden.

Für die erfindungsgemäße Einrichtung ist besonders geeignet ein Hochfrequenz-Generator nach Anspruch 9.

Weitere vorteilhafte Weiterbildungen der Erfindungen sind dem Anspruch 10 zu entnehmen, wobei von besonderer Bedeutung die Ausbildung der Spüllösung als elektrolythaltige Lösung ist, da hierdurch zum einen die Spülflüssigkeit selbst durch Hochfrequenzströme erwärmt wird und auch zur Stromleitung zum Biogewebe wesentlich beiträgt.

Die Erfindung wird im folgenden beispielsweise anhand der Zeichnung beschrieben; in dieser zeigt:
- Fig. 1: eine Ansicht der Arbeitselektrode einer erfindungsgemäßen Hochfrequenz-Thermotherapieeinrichtung und
- Fig. 2: einen vergrößerten Ausschnitt der Arbeitselektrode nach Fig. 1 im distalen Endbereich und in einer geschnittenen Darstellung.

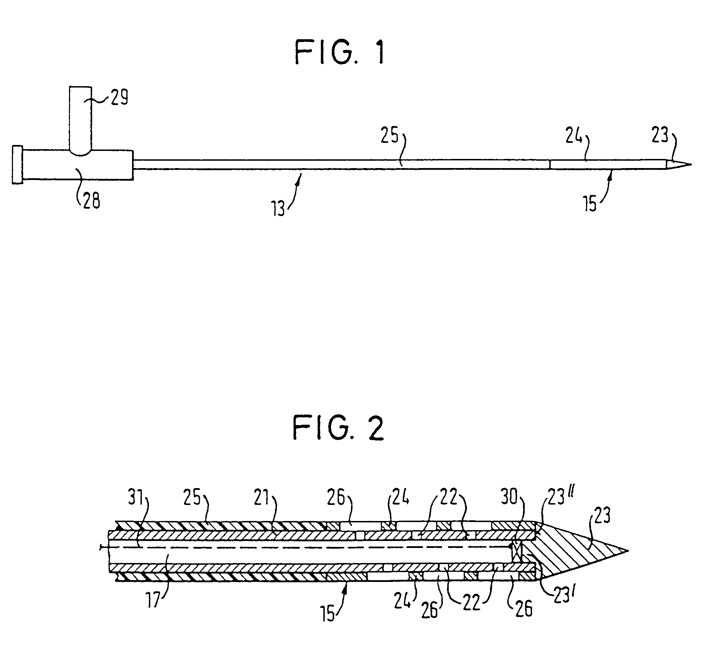

Die Arbeitselektrode 13 einer erfindungsgemäßen Hochfrequenz-Therapieeinrichtung ist nach den Figuren 1 und 2 kanülenartig ausgebildet und weist gemäß Fig. 1 an ihrem proximalen Ende einen Spülflüssigkeitsanschluß 28 sowie einen seitlich abzweigenden Hochspannungsanschluß 29, welcher in nicht dargestellter Weise an einen Hochfrequenzgenerator angeschlossen ist, und im distalen Endbereich eine elektrisch leitfähige Arbeitsspitze 15 auf.

Innen besitzt die Arbeitselektrode 13 nach Fig. 2 ein kanülenartiges Rohr 21, dessen metallische Wandung über den Hochfrequenzspannungsanschluß 29 und eine nicht gezeigte Zuleitung mit der Ausgangsklemme eines Hochfrequenzgenerators verbunden ist, während der im Innern des Rohres 21 ausgebildete Spülkanal- bzw. Flüssigkeitskanal 17 über den Spülflüssigkeitsanschluß 28 und eine nicht dargestellte Schlauchleitung z.B. an eine Infusionspumpe angelegt ist.

Im distalen Endbereich bzw. an der Arbeitsspitze 15 weist das Rohr 21 seitlich Spüllösungsaustrittsöffnungen 22 auf, welche als radiale Mikrobohrungen ausgebildet sind. Nach Fig. 2 sind mehrere derartige Mikrobohrungen 22 über die Länge und den Umfang des Rohres 21 verteilt.

Der distale Endbereich des Rohres 21 ist nach Fig. 1 und 2 von einem zum Rohr 21 konzentrischen Elektrodenrohr 24 umgeben, welches am distalen Ende bündig mit dem Rohr 21 abschließt. Dort ist eine Metallspitze 23 angebracht, welche mit einem hinteren Zapfenteil 23' passend in das Innere des Rohres 21 eingreift und mit einer Ringstufe 23'' an den vorderen Stirnflächen der Rohre 21, 24 anliegt, derart, daß die konusförmige Außenfläche der Metallspitze 23 stufenlos in das kreiszylindrische Elektrodenrohr 24 übergeht. Das Elektrodenrohr 21 und vorzugsweise auch die Metallspitze 23 bestehen aus nichtrostendem, elektrisch leitendem und biokompatiblem Werkstoff, wobei vorzugsweise CrNi-Stähle oder NiTi-Legierungen Verwendung finden.

Hinten an das Elektrodenrohr 24 schließt ein den gleichen Durchmesser und die gleiche Wandstärke aufweisender, auf das Rohr 21 aufgeschrumpfter Isolierschlauch 25 an, der sich nach Fig. 1 bis zum Anschlußstück 28, 29 erstreckt.

Das Elektrodenrohr 24 besitzt nach Fig. 2 Axialschlitze 26, die jeweils mit einer der Mikrobohrungen 22 kommunizieren. Die Mikrobohrungen 22 und die Axialschlitze 26 bilden einen Durchlaß für Spülflüssigkeit mit einer Strömungsgeschwindigkeit von 50 bis 100 ml/h, die durch den Spülkanal 17 mit etwa 0,6 bis 0,7 mm Innendurchmesser von einer nicht gezeigten Infusionspumpe eingespeist wird. Die über den Umfang und die Länge verteilten Axialschlitze 26 im Elektrodenrohr 24 dienen als Spülflüssigkeitsreservoire (Wasserkavernen), die nach dem Einstechen in das Biogewebe einen ungehinderten Spülflüssigkeitsstrom im Einstichkanal um den Zylindermantel des Elektrodenrohres 24, d.h. um die Arbeitsspitze 15 herum garantieren und ein Anhaften sowie Austrocknen während der thermischen Interaktionen mit dem Biogewebe oder Koagulat verhindern. Dadurch wird über lange HF-Applikationszeiten hinweg die elektrolytische Leitfähigkeit im Zielgebiet für HF-Ströme erzielt.

Als Isolierschlauch 25 dient vorzugsweise ein gewebeverträglicher Teflon®-Schlauch, der für eine Dampfsterilisation bis 134°C thermostabil ist und gute Antihaft- und Gleiteigenschaften aufweist. Der Außendurchmesser des Isolierschlauches 25 und des äußeren Elektrodenrohres 24 sind gleich groß.

Da sich das Elektrodenrohr 24 infolge geringer Masse und Wärmekapazität während der thermischen Interaktionen gemeinsam mit dem umgebenden Tumorgewebe und der interstitiellen Spülflüssigkeit erwärmt, kann das Innenlumen 17 des kanülenartigen Rohres 21 zur Temperaturkontrolle mittels eines Mikrothermoelementes 30 benutzt werden, welches nach Fig. 2 unmittelbar hinter der Metallspitze 23 am distalen Ende des Spülkanals 17 angeordnet ist. Von dem Mikrothermoelement 30 verläuft eine gestrichelt angedeutete Meßleitung 31 durch den Spülkanal 17 in das Anschlußstück 28, das mit einem Y-Adapter versehen ist, und von dort in nicht gezeigter Weise zu einem Steuergerät, wo die Temperaturauswertung stattfindet.

Als Mikrothermoelement 30 eignen sich gekapselte Mikrochips oder NiCr-Ni-Mantel-Thermoelemente von 0,25 mm Durchmesser und mit kleinen thermischen Zeitkonstanten von etwa 7 bis 10 ms.

Die Arbeitsweise der beschriebenen Hochfrequenz-Therapieeinrichtung ist wie folgt:

Nachdem der Patient mit einer Neutralelektrode kontaktiert ist und der erfindungsgemäße HF-Nadelapplikator in das Zielgebiet eingebracht wurde, beginnt mit etwa 15 s Zeitverzögerung der Behandlungsprozeß. Nach Einschaltung der Infusionspumpe und des Hochfrequenzgenerators wird die Behandlungsstelle um die Metallspitze 23 herum mit der zum Beispiel aus einer 0,9 %igen NaCl-Lösung bestehenden Spülflüssigkeit umspült und damit in gezielter Weise durchgehend elektrisch leitfähig gemacht. Die thermischen Wirkungen können dabei auch mit nichtelektrolytischen Spüllösungen erzielt werden, da das pathogene Biogewebe sowie das Blut elektrolytisch leitfähig sind. Gleichzeitig fließt von der Metallspitze 23 (Fig. 1, 2) ein Hochfrequenzstrom in den Tumor und erhitzt diesen auf die für die Nekrosebildung erforderliche Temperatur. Dabei erwärmt sich wegen der geringen Wärmekapazität der Rohre 21, 24 sowie der Spitze 23 das Mikrothermoelement 30 (Fig. 2) entsprechend und gibt über die Meßleitung 31 ein entsprechendes Signal an das Steuergerät ab, welches den Hochfrequenzgenerator auf eine vorbestimmte Temperatur am Mikrothermoelement 30 einhaltende Solleistung einregeln kann.

An einem nicht gezeigten Timer kann eine bestimmte Betriebszeit des Hochfrequenzgenerators eingestellt werden. Bevorzugt arbeitet der Hochfrequenzgenerator intervallartig, um in den Intervallpausen störfrei die Temperatursensor-Signale auswerten zu können.

Die erfindungsgemäße Arbeitselektrode 13 besteht vorzugsweise aus einem kanülenförmigen Rohr 21 mit dünnwandiger und temperaturstabiler Isolation 25 von vorzugsweise 200 mm Länge und 1,1 mm Außendurchmesser.

Das Elektrodenrohr 24 soll in Abhängigkeit von der Tumorgröße eine Länge von 10, 20 oder 30 mm aufweisen und steht als Applikator-Set zur Auswahl zur Verfügung.

Die Metallspitze 23 mit einem Spitzenwinkel von etwa 15 Grad oder einem Schrägschliff ist mit den Rohren 21, 24 bevorzugt durch Schweißung, insbesondere Laserschweißung verbunden. Sie gewährleistet eine kraftarme Gewebeverdrängung im Stichkanal und vermeidet Verstopfungen der inneren Metallkanüle 21.

Der Spülkanal 17 des Rohres 21 hat einen Durchmesser von etwa 0,6 bis 0,7 mm, das Mikrothermoelement 30 einen solchen von 0,25 mm und eine geringe thermische Zeitkonstante von 7 bis 10 ms.

Besonders wichtig ist es, daß zur Vermeidung von Hochfrequenz-Störpegelüberlagerungen beim Temperatur-Sensorsignal die Temperaturerfassung vorteilhaft jeweils in den Pausen der bevorzugt intervallartig vorgenommenen Hochfrequenz-Energieapplikation erfolgt. Somit kann auf kostenaufwendige Lichtwellenleiter-Temperatursensoren und Auswertegeräte verzichtet werden.

Mit dem erfindungsgemäßen Gerät werden je nach Energiedosierung, Applikationszeit, Elektrodengeometrie, Volumenstrom der Spüllösung sowie in Abhängigkeit von der Tumorgewebeart einschließlich seiner differenten elektrischen und thermischen Eigenschaften rotationselliptische Koagulationsnekrosen bis zu einem Volumen von 15 cm³ erzielt. Die komplexen Wirkmechanismen der Ausbreitung hochfrequenter elektrischer Felder im Tumorgewebe mit Wärmeentwicklung, Wärmeleitung und Wärmeabtransport durch Blutperfusion bestimmen wesentlich das räumliche Temperaturprofil und das Tumorgewebe-Koagulationsvolumen. Die minimale Invasivität des Verfahrens ermöglicht Mehrfachanwendungen am Patienten in einer oder mehreren Therapiesitzungen bzw. mit einem oder mehreren Nadelapplikatoren.

Eine Echtzeitkontrolle der räumlichen Wärmeausbreitung oder der vollständigen Tumordenaturierung während der Hochfrequenz-Thermotherapie ist mit bekannten Prozeduren der Magnetresonanztomographie möglich, da sich die longitudinale Relaxationszeiten T₁ der Wasserstoffprotonen temperaturspezifisch verändern.

Mit der Computertomographie können die Veränderungen des relativen Schwächungskoeffizienten des erwärmten Biogewebes ausgenutzt werden (Shift von 0,5 bis 1 Hounsfieldeinheiten pro Kelvin). Effektiver erscheint die Kontrolle der Koagulationsausdehnung mittels eines speziellen Regimes der Perfusion und Abbaudynamik von Röntgen-Kontrastmitteln innerhalb und außerhalb des Zielgebietes der Hochfrequenz-Thermotherapie.

Ebenso ist eine Echtzeitkontrolle der Wärmeausbreitung mit bildgebenden Ultraschallgeräten in Verbindung mit Ultraschall-Kontrastmitteln möglich. Erfindungsgemäß ist der Patient Bestandteil eines elektrischen Hochfrequenzstromkreises mit Frequenzen von 300 kHz bis 1 MHz, wobei das pathologische Gewebe durch das kanülenartige und permanent Spülflüssigkeit zuführende Rohr 22, das Elektrodenrohr 24 bzw. die Metallspitze 23 im Interstitium kontaktiert wird. Durch Stromwärmewirkungen von einigen Minuten Dauer wird sowohl die Spüllösung als auch das pathologische Gewebe in der Applikatorumgebung langsam erwärmt. Hierbei wird das Gewebe durch Koagulation devitalisiert.

Der erfindungsgemäße Hochfrequenz-Nadelapplikator ist durch geeignete geometrische Modifikation kompatibel für bekannte Stereotaxievorrichtungen, Biopsievorrichtungen oder Zieleinrichtungen in Verbindung mit bildgebenden Diagnosegeräten (CT, Ultraschall, Röntgen).

### Bezugszeichenliste

- 13: Arbeitselektrode
- 15: Arbeitsspitze
- 17: Flüssigkeitskanal
- 21: kanülenartiges Rohr
- 22: Spüllösungsaustrittsöffnung
- 23: Metallspitze
- 23': Zapfenteil
- 23'': Ringstufe
- 24: Elektrodenrohr
- 25: Isolierschlauch
- 26: Axialschlitz
- 28: Spüllösungsanschluß
- 29: Hochfrequenzspannungsanschluß
- 30: Mikrothermoelement
- 31: Meßleitung

## Patentansprüche

1. Hochfrequenz-Therapieeinrichtung mit einem Nadelapplikator zur interstitiellen Thermotherapie von Tumoren oder pathologischem Gewebe durch lokale thermische Therapie bei Temperaturen im Bereich von 60 bis 100°C mittels Koagulation bzw. Nekrotisierung, mit einer am Körper eines Patienten anbringbaren, vorzugsweise großflächigen Neutralelektrode und einer Arbeitselektrode (13), deren Arbeitsspitze (15) durch eine Einstichöffnung oder natürliche Körperöffnung an den Therapieort bringbar ist sowie mit einem an die Neutralelektrode und die Arbeitselektrode (13) angeschlossenen Hochfrequenzgenerator vorzugsweise mit Konstantleistungscharakteristik und niedriger Ausgangsspannung,
**dadurch gekennzeichnet,**
**daß** die Arbeitselektrode (13) kanülenartig und an ihrem Umfang flüssigkeitsdurchlässig ist und einen axialen Flüssigkeitskanal (17) aufweist, der im Bereich der Arbeitsspitze (15) mündet,
**daß** die Arbeitselektrode (13) ein kanülenartiges Rohr (21) mit metallischer Wandung aufweist, an dessen distalem Endbereich sich - vom proximalen Ende aus gesehen - zunächst radiale Spüllösungsaustrittsöffnungen (22) und dann eine Metallspitze (23) zum Einstechen des Nadelapplikators in Gewebe befinden, wobei die Metallspitze (23) das kanülenartige Rohr (21) an seinem vorderen Ende verschließt, und
**daß** das kanülenartige Rohr (21) auf der von der Metallspitze (23) axial abgewandten Seite der Spüllösungsaustrittsöffnungen (22) bis an den distalen Endbereich mit einem thermostabilen Isolierschlauch (25) oder einer thermostabilen Beschichtung überzogen ist.

2. Einrichtung nach Anspruch 1, **dadurch gekennzeichnet, daß** auf dem distalen Endbereich des kanülenartigen Rohres (21) ein mit diesem konzentrisches, für Spülflüssigkeit am Zylinder-Umfang durchlässiges Elektrodenrohr (24) angeordnet ist, welches an seinem vorderen Ende vorzugsweise mit dem kanülenartigen Rohr (21) und/oder der Metallspitze (23) bündig ist, wobei das Elektrodenrohr (24) vorzugsweise aus nicht rostendem, elektrisch leitendem und biokompatiblem Werkstoff besteht, wobei vorzugsweise CrNi-Stähle oder NiTi-Legierungen Verwendung finden.

3. Einrichtung nach Anspruch 2, **dadurch gekennzeichnet, daß** sich in der Wand des Elektrodenrohres (24) in Ausrichtung mit dem Spüllösungsaustrittsöffnungen (22) mehrere Axialschlitze (26) oder auch Bohrungen befinden, die sich zumindest in axialer Richtung und vorzugsweise beidseits über die Spüllösungsaustrittsöffnungen (22) hinaus erstrecken und als Spülflüssigkeitsreservoire dienen.

4. Einrichtung nach Anspruch 3, **dadurch gekennzeichnet, daß** die Spülflüssigkeitaustrittsöffnungen (22) sowie die Axialschlitze (26) über den Umfang des Rohres (21) verteilt sind, wobei der Bohrungsdurchmesser der Spüllösungsaustrittsöffnungen (22) vorzugsweise 0,4 mm und der Querschnitt der Axialschlitze (26) vorzugsweise 0,4 mm x 4 mm beträgt.

5. Einrichtung nach einem der Ansprüche 2 bis 4, **dadurch gekennzeichnet, daß** die Metallspitze (23) am distalen Ende das kanülenartige Rohr (21) und das Elektrodenrohr (24) verbindet.

6. Einrichtung nach einem der Ansprüche 2 bis 5, **dadurch gekennzeichnet, daß** das Elektrodenrohr (24) in unterschiedlichen Längen von zum Beispiel 10, 20 oder 30 mm zur Verfügung steht, eine entsprechend unterschiedliche Anzahl von Axialschlitzen (26) aufweist und fest mit dem distalen Ende des kanülenartigen Rohres (21) verbunden ist.

7. Einrichtung nach einem der Ansprüche 2 bis 6, **dadurch gekennzeichnet, daß** der thermostabile Isolierschlauch (25) gute Antihaft- und Gleiteigenschaften aufweist und vorzugsweise mit seinem distalen Ende bündig an das Elektrodenrohr (24) anschließt.

8. Einrichtung nach einem der vorhergehenden Ansprüche, **dadurch gekennzeichnet, daß** im distalen Bereich der Arbeitselektrode (13) und vorzugsweise im Spülkanal (17) eine Temperaturmeßanordnung, insbesondere ein Mikrothermoelement (30) angeordnet ist, welches über eine Meßleitung (31) mit einer Temperaturauswerteanordnung verbunden ist.

9. Einrichtung nach einem der vorhergehenden Ansprüche, **dadurch gekennzeichnet, daß** der Hochfrequenzgenerator eine Konstant-Leistungscharakteristik besitzt, wobei die Größe der Leistung vorzugsweise einstellbar ist und so das Koagulationsvolumen wenig abhängig von den elektrischen Leitfähigkeiten des Biogewebes und der Spüllösung gemacht werden kann, wobei insbesondere die Ausgangs-Leistung des Hochfrequenzgenerators gering ist und vorzugsweise zwischen 4 und 40 W liegt sowie insbesondere innerhalb dieses Bereiches oder eines Ausschnittes davon verstellbar ist.

10. Einrichtung nach einem der vorhergehenden Ansprüche, **dadurch gekennzeichnet, daß** die Hochfrequenzspannung zwischen Arbeitselektrode (13) und Patientenkörper unter 140 V_{eff} liegt und einem Mikrolichtbogenfreie Energieübertragung gewährleistet und/oder
daß die Einschaltzeit des im elektrischen Patientenkreis fließenden Hochfrequenzstromes auf eine vorbestimmte Zeitdauer von vorzugsweise einigen Minuten einstellbar ist, und/oder
daß die Spüllösung eine elektrolythaltige Lösung, vorzugsweise eine physiologische Kochsalzlösung, Ringer-Lösung oder ionische Röntgen-Kontrastmittel-Lösung ist, und/oder
daß zur Vermeidung von Tumorzellverschleppungen zelltoxische Medien der Spüllösung beigemischt sind.

## Claims

1. A radio frequency therapy device comprising a needle applicator for the interstitial thermal therapy of tumours or pathological tissue by local thermal therapy at temperatures in the range from 60 to 100°C by means of coagulation or necrotisation, comprising a preferably large area neutral electrode attachable to the body of a patient and a working electrode (13) the working tip (15) of which can be introduced through a penetration opening or a natural body opening at the site of the therapy, and also comprising a radio-frequency generator, preferably with a constant power characteristic and low output voltage, connected to the neutral electrode and to the working electrode (13),
**characterized in that** the working electrode (13) is cannula-like, is permeable to liquid at its periphery and has an axial liquid channel (17) which opens into the region of the working tip (15);
**in that** the working electrode (13) has a cannula-like tube (21) with a metallic wall at the distal end region of which there are located - as seen from the proximal end - firstly radial flushing solution outlet openings (22) and then a metal tip (23) for inserting the needle applicator into tissue, with the metal tip (23) closing the cannula-like tube (21) at its front end;
and **in that** the cannula-like tube (21) is covered at the side of the flushing solution outlet openings (22) axially remote from the metal tip (23) and up to the distal end region with a thermostable insulation hose (25) or a thermostable coating.

2. A device in accordance with claim 1, **characterized in that** an electrode tube (24) which is permeable for flushing liquid at its cylindrical periphery is arranged at the distal end region of the cannula-like tube (21) and is concentric with this, with the electrode tube preferably being flush at its front end with the cannula-like tube (21) and/or the metal tip (23), and with the electrode tube (24) preferably comprising a non-rusting electrically conductive and biocompatible material, with CrNi steels or NiTi alloys preferably being used.

3. A device in accordance with claim 2, **characterized in that** a plurality of axial slots (26) or also bores are located in the wall of the electrode tube (24) in alignment with the flushing solution outlet openings (22), extend at least in the axial direction and preferably on both sides beyond the flushing solution outlet openings (22) and serve as flushing liquid reservoirs.

4. A device in accordance with claim 3, **characterized in that** the flushing liquid outlet openings (22) and also the axial slots (26) are distributed over the periphery of the tube (21), with the bore diameter of the flushing solution outlet openings (22) preferably amounting to 0.4 mm and with the cross-section of the axial slots (26) preferably amounting to 0.4 mm x 4 mm.

5. A device in accordance with any one of claims 2 to 4, **characterized in that** the metal tip (23) connects the cannula-like tube (21) and the electrode tube (24) at the distal end.

6. A device in accordance with any one of claims 2 to 5, **characterized in that** the electrode tube (24) is available in different lengths of, for example, 10, 20 or 30 mm, has a correspondingly different number of axial slots (26) and is fixedly connected to the distal end of the cannula-like tube (21).

7. A device in accordance with any one of claims 2 to 6, **characterized in that** the thermostable insulating hose (25) has good anti-adhesive and sliding characteristics and preferably adjoins the electrode tube (24) in flush manner at its distal end.

8. A device in accordance with any one of the preceding claims, **characterized in that** a temperature measuring device, in particular a microthermal element (30), is arranged in the distal region of the working electrode (13), and preferably in the flushing channel (17), and is connected via a measurement line (31) to a temperature evaluation means.

9. A device in accordance with any one of the preceding claims, **characterized in that** the radio-frequency generator has a constant power characteristic, with the size of the power being preferably adjustable so that the coagulation volume can be made relatively independent of the electrical conductivities of the biological tissue and of the flushing solution, with the output power of the radio-frequency generator in particular being low and preferably lying between 4 and 40 W and also in particular being adjustable within this range or within a section thereof.

10. A device in accordance with any one of the preceding claims,
**characterized in that** the radio-frequency voltage between the working electrode (13) and the patient body lies beneath 140 V_{eff} and ensures a microarc-free energy transmission; and/or **in that** the switched on time of the radio-frequency current flowing in the electrical patient circuit can be set to a predetermined duration of preferably a few minutes; and/or
**in that** the flushing solution is an electrolyte containing solution, preferably a physiological salt solution, a ringer solution or an ionic X-ray contrast agent solution, and/or
**in that** celltoxic media are admixed to the flushing solution to avoid dragging of tumour cells.

## Revendications

1. Appareil à hautes fréquences pour la thermothérapie interstitielle de tumeurs ou de tissu pathologique, à l'aide d'un applicateur à aiguille, par thérapie thermique locale à des températures dans la plage de 60 à 100°C au moyen de coagulation ou de nécrose, comportant une électrode neutre de préférence à grande surface que l'on peut appliquer sur le corps d'un patient, et une électrode de travail (13) dont la pointe de travail (15) peut être amenée au lieu de thérapie à travers un orifice de piqûre ou un orifice corporel naturel, ainsi qu'un générateur à hautes fréquences branché à l'électrode neutre et à l'électrode de travail (13), présentant de préférence une caractéristique de puissance constante et une tension de sortie basse,
**caractérisé en ce que**
l'électrode de travail (13) a l'aspect d'une canule et est perméable aux liquides sur sa périphérie et présente un canal à liquide (17) axial qui débouche dans la région de la pointe de travail (15),
**en ce que** l'électrode de travail (13) présente un tube en forme de canule (21) avec une paroi métallique, dans la région d'extrémité distale duquel, vu depuis l'extrémité proximale, se trouvent tout d'abord des orifices de sortie (22) d'une solution de rinçage et ensuite une pointe métallique (23) pour planter l'applicateur à aiguille dans le tissu, la pointe métallique (23) fermant le tube en forme de canule (21) à son extrémité antérieure, et
**en ce que** sur le côté des orifices de sortie (22) de la solution de rinçage, qui est détourné axialement de la pointe métallique (23), le tube en forme de canule (21) est recouvert, jusqu'à la région d'extrémité distale, avec un tuyau isolant (25) thermostable ou avec un revêtement thermostable.

2. Appareil selon la revendication 1, **caractérisé en ce que** sur la région d'extrémité distale du tube en forme de canule (21) est agencé un tube à électrode (24) concentrique à celui-ci et perméable au liquide de rinçage sur sa périphérie cylindrique, tube à électrode qui est en affleurement à son extrémité antérieure de préférence avec le tube en forme de canule (21) et/ou avec la pointe métallique (23), le tube à électrode (24) étant de préférence en un matériau insensible à la corrosion, conducteur de l'électricité et biocompatible, pour lequel on utilise de préférence des aciers chrome/nickel ou des alliages nickel/titane.

3. Appareil selon la revendication 2, **caractérisé en ce que** dans la paroi du tube à électrode (24) et en alignement avec les orifices de sortie (22) de la solution de rinçage se trouvent plusieurs fentes axiales (26) ou bien des perçages qui s'étendent au moins en direction axiale et de préférence des deux côtés au-delà des orifices de sortie (22) de la solution de rinçage et qui servent de réservoir de liquide de rinçage.

4. Appareil selon la revendication 3, **caractérisé en ce que** les orifices de sortie (22) de la solution de rinçage ainsi que les fentes axiales (26) sont réparties sur la périphérie du tube (21), le diamètre de perçage des orifices de sortie de la solution de rinçage (22)étant de préférence de 0,4 mm et la section transversale des fentes axiales (26) étant de préférence de 0,4 mm x 4 mm.

5. Appareil selon l'une des revendications 2 à 4, **caractérisé en ce que** la pointe métallique à l'extrémité distale relie le tube en forme de canule (21) et le tube à électrode (24).

6. Appareil selon l'une des revendications 2 à 5, **caractérisé en ce que** le tube à électrode (24) est disponible à des longueurs différentes de par exemple 10, 20 ou 30 mm, **en ce qu'**il présente, de manière correspondante, un nombre différent de fentes axiales (26), et **en ce qu'**il est relié solidairement à l'extrémité distale du tube en forme de canule (21).

7. Appareil selon l'une des revendications 2 à 6, **caractérisé en ce que** le tuyau isolant thermostable (25) présente de bonnes propriétés d'anti-adhérence et de glissement et se raccorde de préférence par son extrémité distale en affleurement avec le tube à électrode (24).

8. Appareil selon l'une des revendications précédentes, **caractérisé en ce que** dans la région distale de l'électrode de travail (13) et de préférence dans le canal de rinçage (17) est agencé un système de mesure de température, en particulier un élément micro-thermique (30) qui est relié à un système d'analyse de température via une conduite de mesure (31).

9. Appareil selon l'une des revendications précédentes, **caractérisé en ce que** le générateur à hautes fréquences possède une caractéristique de puissance constante, la valeur de la puissance étant de préférence réglable et le volume de coagulation pouvant ainsi être rendu peu dépendant des conductivités électriques du tissu biologique et de la solution de rinçage, en particulier la puissance de sortie du générateur à hautes fréquences étant faible et située de préférence entre 4 et 40 W et cette puissance de sortie étant en particulier réglable dans cette plage ou dans un secteur de celle-ci.

10. Appareil selon l'une des revendications précédentes, **caractérisé en ce que** la tension à hautes fréquences entre l'électrode de travail (13) et le corps du patient est inférieure à 140 V_{eff} et assure une transmission d'énergie exempte de micro-arcs et/ou **en ce que** la durée de fonctionnement du courant à hautes fréquences s'écoulant dans le cuit électrique du patient est réglable à une durée prédéterminée et de préférence à quelques minutes, et/ou
**en ce que** la solution de rinçage est une solution contenant de l'électrolyte, de préférence une solution de sérum physiologique, de solution de Ringer, ou une solution ionique de substance de contraste radiologique,
et/ou
**en ce que** des agents toxiques aux cellules sont mélangés à la solution de rinçage pour éviter des transmissions de cellules tumorales.
